# EUROPEAN PATENT SPECIFICATION

(11) **EP 2 594 221 B1**
(45) Date of publication and mention of the grant of the patent: **18.10.2017**
(21) Application number: 12192919.4
(22) Date of filing: 16.11.2012
(51) Int. Cl.: A61B 90/00, A61B 90/13, A61B 17/00

(54) **In-situ proximity recognition apparatus**
In-situ-Näherungserkennungsvorrichtung
Appareil de reconnaissance de proximité in situ

(30) Priority: 18.11.2011 US 201161561482 P; 09.10.2012 US 201213647484
(43) Date of publication of application: 22.05.2013
(73) Proprietor: Covidien LP, Mansfield, MA 02048 (US)
(72) Inventor: Sharonov, Alexey, Bethany, CT 06524 (US)
(74) Representative: Soames, Candida Jane

(56) References cited:
- WO-A1-02/15973
- WO-A1-98/38941
- WO-A2-2011/064768
- DE-A1- 19 830 183
- US-A1- 2002 077 542
- US-A1- 2005 020 909

## Description

### BACKGROUND

### 1. Technical Field

The present disclosure relates to systems for estimating the proximity of a medical device to a target location inside a patient.

### 2. Background of the Related Art

During minimal invasive surgery, it is often important to know where a surgical tool or internal features are located. Surgeons can have a difficult time locating a desired location inside a patient to accurately operate on or position an instrument in the patient. Devices exist that can help in situ location of instruments, but they require emitters to be present on the medical device itself. This requires the use of potentially toxic batteries or powerful magnets for the medical device to generate a signal that can be read. A way to indicate position with respect to a desired location inside a patient without the use of emitter on the medical device would lead to a safer more cost effective device.

WO2011/064768A describes a device for accurate dental implant fixture location determination including an inductive eddy current effect based dental implant fixture location sensor and a handle.

WO98/38941 describes various receiver/transmitter configurations for positioning a blood vessel graft relative to a blood vessel in the heart.

US2002/0077542A describes systems for navigation-assisted dental treatment.

DE19830183A describes a temperature measurement device having a navigation element near the tip of the device that interacts with an inhomogeneous magnetic field to provide position information.

US2005/0020909A describes a display unit for use with a surgical navigation apparatus. In one embodiment the display element may change the color intensity or brightness of certain display elements to show that a proper position and/or orientation has been achieved.

### SUMMARY

The present invention provides a proximity sensing system for determining proximity to a desired location inside a patient, comprising: an emitter assembly comprising: at least one power supply; and at least one coil operably coupled to said at least one power supply and configured to selectively output electromagnetic energy; characterized in that said proximity sensing system further comprises a medical device comprising: an elongate member including a distal portion and a proximal portion; at least one adapter integrally or removably connected to and positioned on said distal portion, said at least one adapter comprising at least one inductor configured to inductively couple to said coil and convert electromagnetic energy into an electrical current; and at least one indicator disposed integrally or removably upon the adapter wherein said at least one indicator is operably coupled to said inductor and comprises a visual indicator configured to convert said electrical current into light having increasing intensity as the adapter is moved closer to the coil.

The at least one visual indicator may be at least one light-emitting diode (LED).

In accordance with at least one aspect of the present disclosure, the adapter and indicator may be configured to selectively secure to the elongate member and to be selectively removable therefrom.

The systems of the present invention may be used in a method of determining the proximity of a medical device to a desired location inside a patient.

The method comprises placing the at least one coil outside of the patient over the desired location, allowing electrical current to pass through the at least one coil to create electromagnetic energy, positioning the distal portion of the medical device inside the patient, receiving electromagnetic energy from the coil using the inductor thereby inductively coupling the inductor to the coil to create electrical current in the inductor, and powering the indicator using electrical current from the inductor.

The method may further include determining an intensity of the indicator when the medical device is in a first position relative to the coil and a second position relative to the coil, wherein the intensity is proportional with respect to the relative positioning of the coil and the medical device.

The method may further include comparing the intensity at the first position with the intensity at the second position to determine if the medical device has moved closer or further from the desired location.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1A is a perspective view of an embodiment of medical device having an elongate member in accordance with this disclosure;
Fig. 1B is a perspective view of a distal portion of the elongate member of Fig. 1A, in accordance with this disclosure;
Fig. 2 is a perspective view of an embodiment of an elongate member in accordance with this disclosure;
Fig. 3 is a view of a system in accordance with this disclosure; and
Fig. 4 is a circuit diagram of an inductor operably coupled to a diode in accordance with this disclosure.

### DETAILED DESCRIPTION

Particular embodiments of the present disclosure are described hereinbelow with reference to the accompanying drawings; however, the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus that is closer to the user and the term "distal" refers to the end of the apparatus that is farther away from the user. The term "clinician" refers to any medical professional (e.g., doctor, surgeon, nurse, or the like) performing a medical procedure involving the use of embodiments described herein.

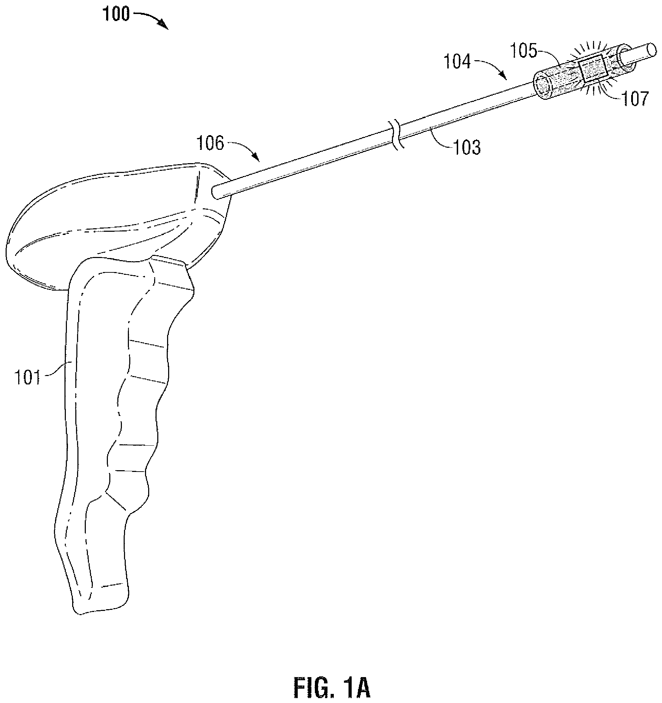

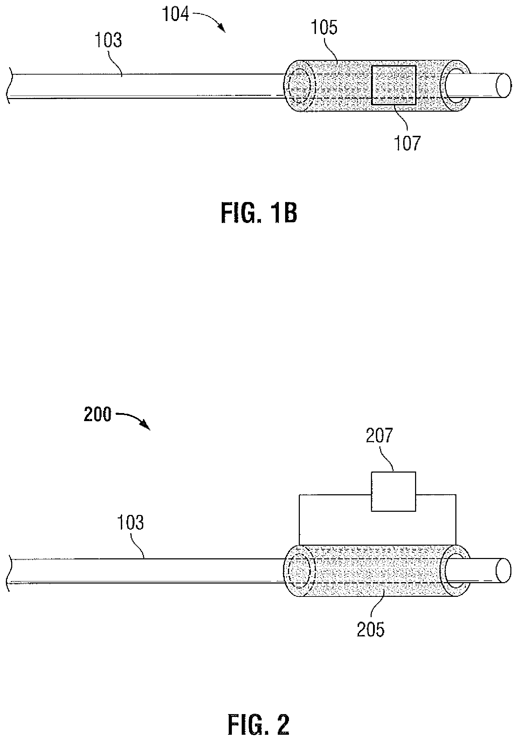

In accordance with at least one aspect of the present disclosure, a medical device is described herein. With reference to Fig. 1A and 1B, the medical device 100 may have an elongate member 103 including a proximal portion 106 and a distal portion 104. The elongate member 103 may be integrally or removably coupled to a housing 101. Housing 101 may be operably coupled to an end effector disposed on the elongate member 103. Examples of end effectors include but are not limited to vessel sealing devices, linear stapling devices, circular stapling devices, cutters, etc. Furthermore, an end effector may be configured to deploy a probe, implant, or other device in a desired condition.

At least one adapter 105 is operably coupled to the elongate member 103. The operable coupling between adapter 105 and elongate member 103 may be any suitable integral or removable connection, such as but not limited to a latch, an adhesive, and a contact friction connection.

The adapter 105 includes at least one inductor configured to convert electromagnetic energy into an electrical current. The at least one inductor may be any device suitable for converting electromagnetic (EM) energy into electrical current, such as but not limited to at least one wire coil, at least one antenna configured to accept at least one desired EM frequency, and combinations thereof. The inductor is configured to inductively couple and accept EM energy from an emitter, thereby creating an electrical current having an intensity proportional to the distance from the emitter. For example, the intensity may increase as distance from the emitter decreases.

Adapter 105 may simply be a wire coil inductor that is either integrally or removably connected to the elongate member 103. The at least one adapter 105 may be connected integrally upon the distal portion 104 such that it forms at least a portion of the elongate member 103. In some embodiments, the at least one adapter 105 is removably connected to the distal portion 104 such that the adapter 105 may be replaced as desired. For example, the adapter 105 may be able to slide on to the elongate member 103 and selectively secure to the elongate member 103, and then further be releasable such that adapter 105 may slide off of the elongate member 103.

The medical device 100 and/or adapter 105 include at least one indicator 107 operably coupled to the inductor in adapter 105 such that inductor 105 and indicator 107 are in a permanent or selectable electrical connection. As shown in Fig. 1B, indicator 107 is disposed integrally or removably upon the adapter 105.

In some embodiments, the at least one indicator 107 is integrally connected to the elongate member 103. However, the indicator 107 may also be removably connected to the elongate member 103 so as to allow removal or replacement of indicator 107. In some embodiments, both the adapter 105 and indicator 107 may be configured to selectively secure to the elongate member 103 and to be selectively removable therefrom.

The at least one indicator 107 is at least one visual indicator configured to convert the electrical current produced by the inductor(s) in adapter 105 into visible or invisible light. The light produced may be of any desired wavelength range including but not limited to visible light, RF, microwave, ultraviolet, and infrared. For example, where the indicator 107 emits a visible light, the user may look directly at the light source to determine intensity. Where the indicator 107 emits an invisible frequency of light, the user may use a detector to sense the invisible light emission and intensity thereof.

In at least some embodiments, the at least one visual indicator is at least one light-emitting diode (LED). The at least one LED may be of any color, size, or shape desired. In some embodiments, the at least one LED has a first color in a first condition and a second color at a second condition.

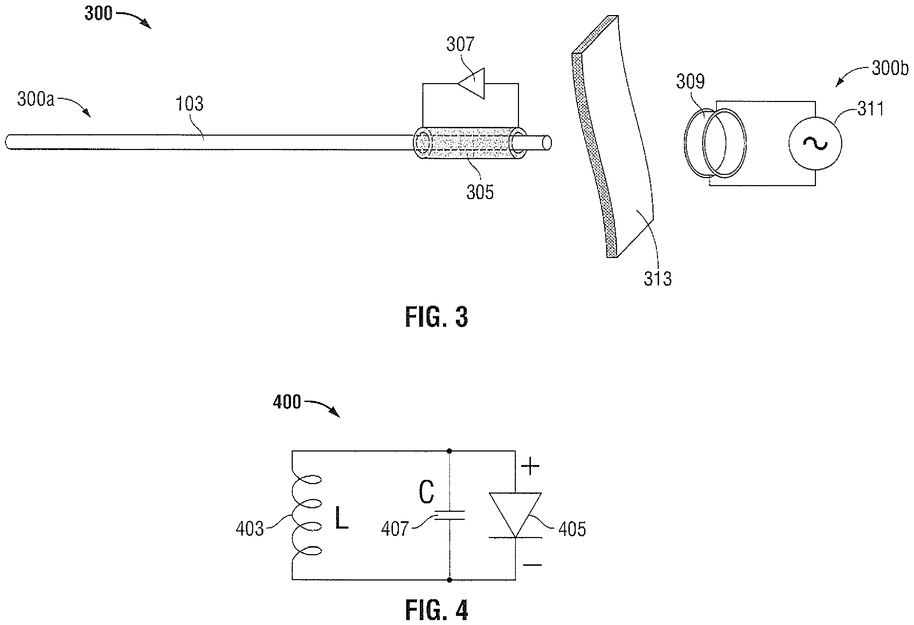

In some embodiments, multiple LED's may be present having the same or a combination of colors, shapes, and sizes. For example, a first LED of a first color may operate a first range of voltages/currents and a second LED of a second color may operate at second range of voltages/currents. Such color cascading may be extrapolated to any number of LED's and current ranges. Referring to Fig. 4 which shows a circuit diagram of an adapter-indicator assembly 400, inductor 403 is connected to diode 405. At least one capacitor 407 may also be disposed in parallel with the inductor 403 and the diode 405.

Referring back to Fig. 1A and 1B, using a visual indicator allows the medical device 100 to produce light of increasing intensity as the adapter 105 is moved closer to an emitter because the inductor produces increasing current with decreasing distance to the emitter. Thus, the user may approximate the position of the medical device 100 relative to the emitter by viewing or measuring the intensity of the light. Furthermore, in embodiments where a plurality of colored light sources exists, the user may approximate the position of the medical device 100 relative to the emitter by viewing a change in the color of the activated light sources.

In some embodiments, the light may also be pulsed relative to the amount of current being produced by the inductor. For example, indicator 105 may comprise a light configured to pulse with increasing frequency proportional to the inductively produced current such that the closer the adapter 105 is positioned to a desired location, the more frequently the light blinks. Thus, the user may approximate the position of the medical device 100 relative to the emitter by viewing or measuring the blink frequency of the light.

Referring to Fig. 3, the system 300 has at least one medical device 300A having an elongate member 103 including a distal portion, a proximal portion, an adapter 305, and an indicator 307. The medical device 300A, the adapter 305, and the indicator 307 may be as any other described above.

System 300 further has at least one emitter assembly 300B comprising at least one power supply 311, at least one emitter 309 operably coupled to the at least one power supply 311 and configured to selectively output electromagnetic energy. In some embodiments, the power supply 311 is an electro-surgical generator, but the generator may be any suitable source of power such as but not limited to one or more batteries or other common power sources. The power supply 311 may provide alternating current (AC) or direct current (DC).

In some embodiments, emitter 309 may comprise at least one coil that may produce a desired magnetic field when current is passed therethrough. Emitter 309 may also be an antenna configured to produce electro-magnetic radiation when current is passed therethrough.

When power supply 311 and emitter 309 are electrically coupled, a current passes through the emitter causing an electro-magnetic field to be created as a function of the current. For a coil emitter, a magnetic field is created around the coil having an intensity related to the amount of current passing through the coil. If an electromagnetic field is present, the inductor in adapter 305 can convert the EM field back into electrical current if the adapter 305 is placed within range of the EM field. The closer the adapter 305 is placed to the emitter 309, the larger the voltage drop and the stronger the current the inductor will produce. The current produced by the inductor can be directed to the indicator 307, either directly or through one or more circuit components such as but not limited to AC-to-DC converters, signal conditioning stages, and amplifiers. Furthermore, medical device 300A may have an end effector designed to manually or automatically deploy a probe, implant, or other device when the produced current reaches a certain threshold.

A method of determining the proximity of a medical device to a desired location inside a patient using the systems of the invention is also herein disclosed. The desired location may be a tissue, vessel, or organ inside the patient chosen by a clinician to accomplish a desired medical procedure such as, but not limited to minimally invasive surgery.

The method includes providing at least one power supply 311 as described above, at least one emitter 309 as described above operably coupled to the at least one power supply 311 and configured to selectively output electromagnetic energy. The method may also include providing at least one medical device 300A as described above having a elongate member 103 comprising a distal portion, a proximal portion and at least one adapter 305 as described above including at least one inductor as described above operably coupled to the distal portion and configured to inductively couple to the at least one emitter 309 and convert electromagnetic energy into electrical current. At least one indicator 307 as described above that is operably connected to the inductor is be provided.

The method may further include placing the at least one emitter 309 outside of the patient over the desired location, as is shown in Fig. 3. Referring to Fig. 3, emitter 309 is placed on the outside of the patient near a desired location and medical device 300A is at least partially disposed within the patient and separated from emitter assembly 300B by tissue 313. For example, if a clinician is attempting to locate an organ in the abdomen of the patient, then the clinician may place the emitter 309 on the patient's abdomen approximately where the location of the organ is or should be.

The method further includes allowing electrical current to pass through the at least one emitter 309 to create an electromagnetic field. In the case where the emitter 309 is a coil, a magnetic field is created when current is passed through the coil. The method may further include positioning the distal portion of the medical device 300A at least partially inside the patient, as shown in Fig. 3. The user may enter a body cavity through an incision in the patient's tissue and position the distal portion as desired.

The method further includes receiving electromagnetic energy from the emitter 309 using the inductor in the adapter 305 thereby inductively coupling the inductor to the emitter to create electrical current in the inductor. For example, after having positioned the distal portion of the medical device inside the patient, the user may generally attempt to find the EM field being emitted by the emitter 309 in order to receive energy using the inductor.

The method further includes powering the indicator 307 using electrical current from the inductor. When the user positions the adapter 305 into the EM field created by the emitter 307, at least a portion of the received energy is converted into electrical energy for powering at least the indicator(s). In some embodiments, the indicator 307 is powered solely from the current created by the inductor, but it is possible to use power from other sources for the indicator 307.

The method may further include determining an intensity of the indicator 307 when the medical device 300A is in a first position relative to the emitter 309 and a second position relative to the emitter 309. The intensity proportionally corresponds to relative position of the adapter 305 from the emitter 309 and, thus, to the desired location. For example, a clinician may determine or measure the brightness or frequency of blinking of the indicator 307 as a function of location of the distal portion of the medical device 300A.

The method may further include comparing the intensity at the first position with the intensity at the second position to determine if the medical device 300A has moved closer or further from the desired location. For example, a clinician may compare the brightness of two or more positions to determine the proximity of the distal portion of the medical device 300A to the desired location.

When provided with a medical device as described above, a clinician may operate on a patient using the medical device as either just a locating tool or for other surgical means. When used as a locating tool, the device allows the clinician to find an in situ location to a higher level of precision which can allow for further insertion of other medical instruments at that location.

It should be understood that the foregoing description is only illustrative of the present disclosure. Various alternatives and modifications can be devised by those skilled in the art without departing from the invention as defined in the accompanying claims.

## Claims

1. A proximity sensing system (300) for determining proximity to a desired location inside a patient, comprising:
an emitter assembly (300b) comprising:
at least one power supply (311); and
at least one coil (309) operably coupled to said at least one power supply and configured to selectively output electromagnetic energy; wherein said proximity sensing system further comprises a medical device (300a) comprising:
an elongate member (103) including a distal portion (104) and a proximal portion (106); and at least one adapter (105,305) integrally or removably connected to and positioned on said distal portion (104), said at least one adapter comprising at least one inductor (403) configured to inductively couple to said coil and convert electromagnetic energy into an electrical current; **characterised in that** said proximity sensing system further comprises at least one indicator (107,307,405) disposed integrally or removably upon the adapter (105,305) wherein said at least one indicator is operably coupled to said inductor and comprises a visual indicator configured to convert said electrical current into light having increasing intensity as the adapter (105,305) is moved closer to the coil.

2. The system of claim 1, wherein said indicator (107,307,405) is at least one LED.

3. The system of claim 1, wherein said adapter (105,305) and indicator (107,307,405) are configured to selectively secure to said elongate member (103) and to be selectively removable therefrom.

## Patentansprüche

1. Näherungserfassungssystem (300) zum Bestimmen der Nähe zu einem gewünschten Ort innerhalb eines Patienten, umfassend:
eine Emitteranordnung (300b) umfassend:
zumindest eine Stromversorgung (311); und
zumindest eine Wicklung (309), die betriebsmäßig mit der zumindest einen Stromversorgung gekoppelt und dazu konfiguriert ist, um selektiv elektromagnetische Energie auszugeben;
wobei das Näherungserfassungssystem weiter eine medizinische Vorrichtung (300a) umfasst, die umfasst:
ein langgestrecktes Element (103), das einen distalen Abschnitt (104) und einen proximalen Abschnitt (106) umfasst; und
zumindest einen Adapter (105, 305), der integral oder entfernbar mit dem distalen Abschnitt (104) verbunden und an diesem positioniert ist, wobei der zumindest eine Adapter zumindest eine Induktionsspule (403) umfasst, die dazu konfiguriert ist, um sich induktiv mit der Wicklung zu koppeln und elektromagnetische Energie in einen elektrischen Strom umzuwandeln, **dadurch gekennzeichnet, dass** das Näherungserfassungssystem weiter zumindest einen Anzeiger (107, 307, 405) umfasst, der integral oder entfernbar an dem Adapter (105, 305) angeordnet ist, wobei der zumindest eine Anzeiger betriebsmäßig mit der Induktionsspule gekoppelt ist und eine Anzeige umfasst, die dazu konfiguriert ist, um den elektrischen Strom in Licht umzuwandeln, das eine zunehmende Intensität aufweist, wenn der Adapter (105, 305) näher an die Wicklung bewegt wird.

2. System nach Anspruch 1, wobei der Anzeiger (107, 307, 405) zumindest eine LED ist.

3. System nach Anspruch 1, wobei der Adapter (105, 305) und Anzeiger (107, 307, 405) dazu konfiguriert sind, um selektiv an dem langgestreckten Element (103) gesichert zu werden und um selektiv davon entfernbar zu sein.

## Revendications

1. Système de détection de proximité (300) pour déterminer la proximité d'une localisation souhaitée à l'intérieur d'un patient, comprenant :
un ensemble émetteur (300b) comprenant :
au moins une alimentation en énergie (311) ; et
au moins une bobine (309) couplée de manière opérationnelle à ladite au moins une alimentation en énergie et configurée pour sortir de manière sélective de l'énergie électromagnétique ;
dans lequel ledit système de détection de proximité comprend en outre un dispositif médical (300a) comprenant :
un élément allongé (103) incluant une portion distale (104) et une portion proximale (106) ;
et
au moins un adaptateur (105, 305) relié d'un seul tenant ou de façon amovible à et positionné sur ladite portion distale (104), ledit au moins un adaptateur comprenant au moins une bobine d'inductance (403) configurée pour un couplage par induction à ladite bobine et pour transformer l'énergie électromagnétique en un courant électrique ; **caractérisé en ce que** ledit système de détection de proximité comprend en outre
au moins un indicateur (107, 307, 405) disposé d'un seul tenant ou de façon amovible sur l'adaptateur (105, 305), dans lequel ledit au moins un indicateur est couplé de manière opérationnelle à ladite bobine d'inductance et comprend un indicateur visuel configuré pour transformer ledit courant électrique en lumière ayant une intensité croissante lorsque l'adaptateur (105, 305) est rapproché de la bobine.

2. Système selon la revendication 1, dans lequel ledit indicateur (107, 307, 405) est au moins une DEL.

3. Système selon la revendication 1, dans lequel ledit adaptateur (105,305) et l'indicateur (107, 307, 405) sont configurés pour fixation de manière sélective audit élément allongé (103) et pour être amovibles de manière sélective de ce dernier.
